# EUROPEAN PATENT SPECIFICATION

(11) **EP 3 402 413 B1**
(45) Date of publication and mention of the grant of the patent: **06.08.2025**
(21) Application number: 17703510.2
(22) Date of filing: 13.01.2017
(51) Int. Cl.: A61B 17/00

(54) **VASCULAR CLOSURE SYSTEM WITH INTRODUCER FOR SHEATH TRANSFER**
GEFÄSSVERSCHLUSSSYSTEM MIT EINFÜHRINSTRUMENT FÜR SCHLEUSENTRANSFER
SYSTÈME DE FERMETURE VASCULAIRE AVEC DISPOSITIF D'INTRODUCTION POUR TRANSFERT DE GAINE

(30) Priority: 13.01.2016 US 201662278298 P
(43) Date of publication of application: 21.11.2018
(73) Proprietor: Teleflex Life Sciences LLC, Wilmington, DE 19808 (US)
(72) Inventor: WALTERS, Greg, A., Exton, PA 1934 (US); GRINTZ, Joseph, Todd, Glenmoore, PA 19343 (US)
(74) Representative: Haseltine Lake Kempner LLP
(86) International application number: PCT/US2017/013314
(87) International publication number: WO 2017/123853

(56) References cited:
- WO-A1-2015/099977
- US-A1- 2014 309 686
- US-A1- 2014 364 899

## Description

### CROSS-REFERENCE TO RELATED APPLICATIONS

The present application claims the benefit and priority to U.S. Provisional Application Serial No. 62/278,298, filed January 13, 2016.

### TECHNICAL FIELD

The present disclosure relates to vascular closure system with an introducer configured for sheath transfer.

### BACKGROUND

During an interventional cardiovascular procedure a puncture may be made in the femoral artery. Advanced cardiovascular procedures may obtain access to the aorta via the vena cava in situations where the femoral artery is not a suitable approach path. In one example, the procedure is a trans-caval aortic valve replacement procedure, or "trans-caval" procedure. Vascular closure devices composed of an absorbable intra-arterial toggle, an extra-vascular folding sealing plug, and a connecting suture, such as a filament, have been developed and may be used to seal these punctures. These devices function by compressing the intra and extraarterial components together around the puncture, with sufficient tension within the connecting suture. However, as the size of percutaneous sheaths become larger to accommodate larger cardiovascular devices, the size of the resulting puncture increases. Larger punctures are harder to seal because of the larger vessel wall defect or puncture. In the case of sealing blood pressure with an external plug, larger defects expose the plug to increased forces, which must be supported through the connecting suture by the intra-arterial toggle. WO2015/099977 A1 describes a vascular closure device and an introducer/procedure access sheath for use with the device. The vascular closure device including a release component, a delivery component, a sealing device and at least one actuator. The release component is elongate along a longitudinal direction, and defines a distal end and a proximal end. The delivery component extends along the release component such that at least the release component is movable relative to the delivery component. The delivery component includes a delivery tube body and defines a delivery tube channel. The sealing device has a toggle that is at least partially disposed within the release tube, a suture that is attached to the toggle and extends through the delivery tube channel, and a plug that is attached to the suture proximal to the toggle. The actuator is coupled to the release component and is in communication with the suture such that actuation of the actuator causes (i) the release component to move the proximal direction relative to the delivery component so as to release the toggle from the release component, and (ii) the suture to be pulled in a proximal direction to thereby place the filament in tension and urge the toggle against a distal end of the delivery component such that the toggle is oriented in a sealing position. US 2014/309686 A1 describes an apparatus for delivering a closure element, including an introducer sheath, a locator and an actuator assembly. US 2014/364899 A1 discloses systems and methods for closing vascular access ports.

### SUMMARY

An embodiment of the present disclosure is a system configured to seal a puncture in a vessel according to claim 1. Optional features are defined in the dependent claims.

The use of the introducer limits blood loss during an exchange of a procedure access sheath used to guide a catheter (or other medical device) into the vessel, e.g. the femoral artery or the aorta, with an access sheath for use with a vascular closure device.

### BRIEF DESCRIPTION OF THE DRAWINGS

The foregoing summary, as well as the following detailed description of an exemplary embodiments of the application, is better understood when read in conjunction with the appended figures. The figures illustrate exemplary embodiments for the purposes of illustration. It should be understood, however, that the application is not limited to the precise arrangements and systems shown.
Figure 1 is a perspective view of a vascular closure system in accordance with an embodiment of the present disclosure;
Figure 2A is a perspective view of an introducer and an access sheath of the system shown in Figure 1;
Figure 2B is a perspective view of the vascular closure device and access sheath of the system shown in Figure 1;
Figure 3 is a side view of the introducer shown in Figure 1;
Figure 4A is a side view an introducers according to an embodiment of the present disclosure;
Figure 4B is a side view an introducers according to another embodiment of the present disclosure;
Figure 4C is a side view an introducers according to another embodiment of the present disclosure;
Figure 5A is a perspective view of a vascular closure device in accordance with an embodiment of the present disclosure;
Figure 5B is a partial cut-away view of the vascular closure device shown in Figure 5A;
Figure 5C is a perspective view of a sealing device associated with the vascular closure device in Figure 5A;
Figure 5D is a side sectional view of a distal portion of the vascular closure device;
Figures 6A-6C are rear perspective views of the vascular closure device with portions of the device removed for clarity;
Figure 6D is a sectional view of the vascular closure device shown in Figure 6A;
Figure 6E is a perspective view of the release component, delivery component, and tensioner of the vascular closure device shown in Figure 6A;
Figure 6F is a perspective cross-sectional view of the a release component, delivery component, and a tensioner of the vascular closure device shown in Figure 6E, taken along line 6F-6F;
Figure 6G is a perspective view of the delivery component and tensioner of the vascular closure device shown in Figure 6E;
Figures 6H and 6I are perspective and top views, respectively, of the release component of the vascular closure device shown in Figure 5A;
Figure 7A is a schematic showing an access sheath from a cardiovascular surgical procedure partially disposed within a vessel through puncture sites in a vessel;
Figure 7B is a schematic showing the introducer from Figure 1 inserted in the access sheath shown in Figure 7A;
Figure 7C is a schematic showing the removal of the access sheath from the vessel with the introducer remaining in position;
Figure 7D is a schematic showing the access sheath of the system shown in Figure 1 inserted into the vessel along the introducer sheath;
Figure7E is a schematic showing the introducer removed from the access sheath and the vascular closure device positioned for insertion into the access sheath;
Figure 7F is a schematic showing the sealing element fully sealing the puncture site.

### DETAILED DESCRIPTION OF ILLUSTRATIVE EMBODIMENTS

Certain terminology is used in the following description for convenience only and is not limiting. The words "right", "left", "lower" and "upper" designate directions in the drawings to which reference is made. The words "proximally" and "distally" refer to directions toward and away from, respectively, the individual operating the system. The terminology includes the above-listed words, derivatives thereof and words of similar import.

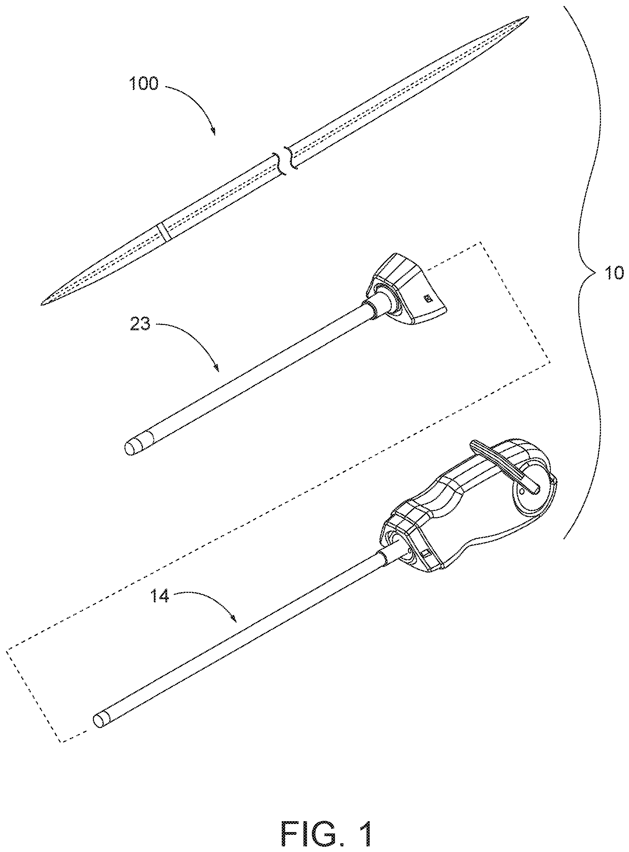

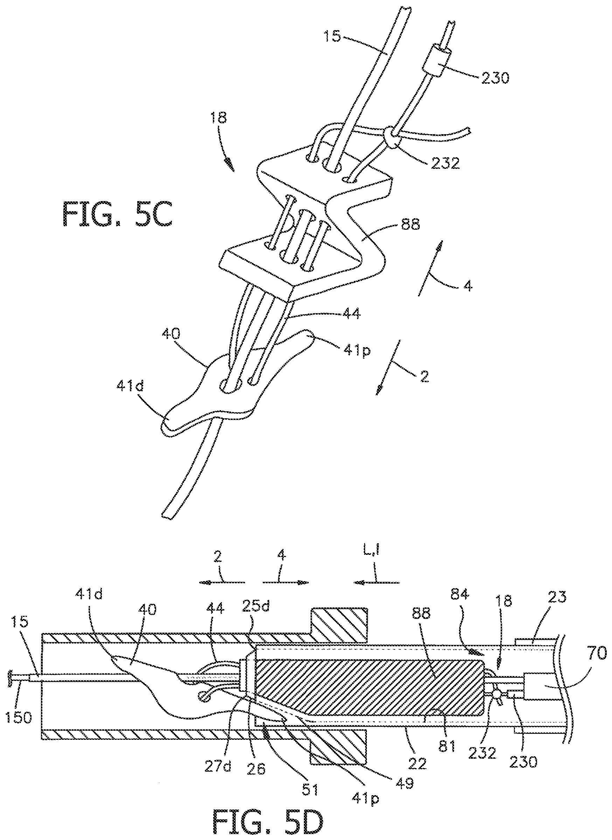

Referring to Figures 1-2B, the vascular closure system 10 includes an introducer 100 and a closure device 12 that is configured to seal the puncture in a vessel wall. The introducer 100 is configured to facilitate placement of the closure device 12 into the desired position within a puncture site of a vessel wall following a surgical procedure. The closure device 12 includes a deployment assembly 14 and an access sheath 23. The access sheath 23 can be inserted into the vessel and the deployment assembly 23 can be inserted into the access sheath 23 to position a sealing device or implantable unit 18 (Figure 5C) into the vessel. The access sheath 23 and introducer 100 can be referred to as insertion assembly 15.

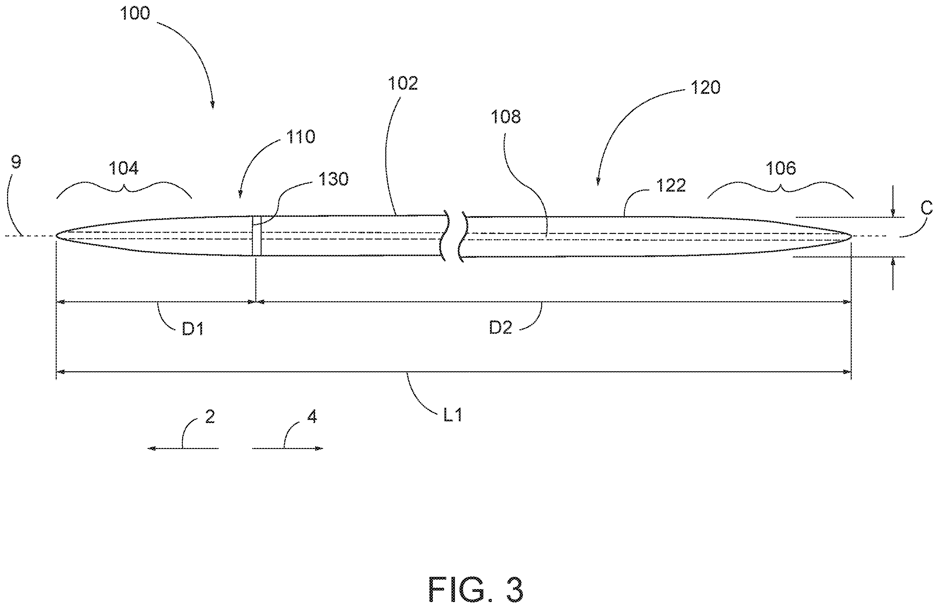

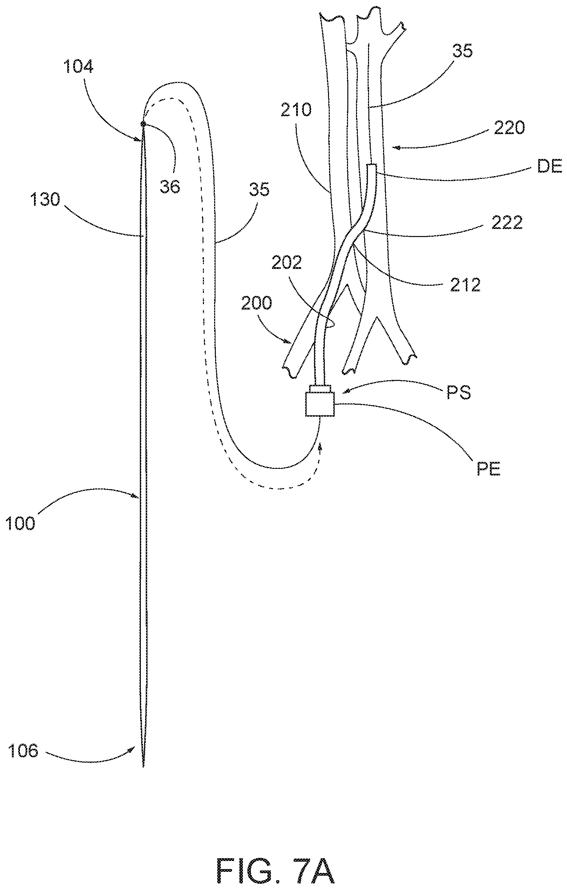

Figures 3-4B illustrates the embodiments of an introducer 100. The introducer 100 is configured to be inserted through the puncture along a guidewire 35 (Figure 7A) that extends through the puncture into the vessel. The introducer 100 includes an introducer body 102 that is elongate along a central axis 9, a proximal tapered tip 106 a distal tapered tip 104 that is opposite to the proximal tapered tip 106 along the central axis 9, and a bore 108 that extends from the proximal tapered tip 106 to the distal tapered tip 104 along the central axis 9. The bore is sized to receive the guidewire 35 (not shown) therethrough. The distal tapered tip of the introducer is configured to be inserted into the vessel along the guidewire 35.

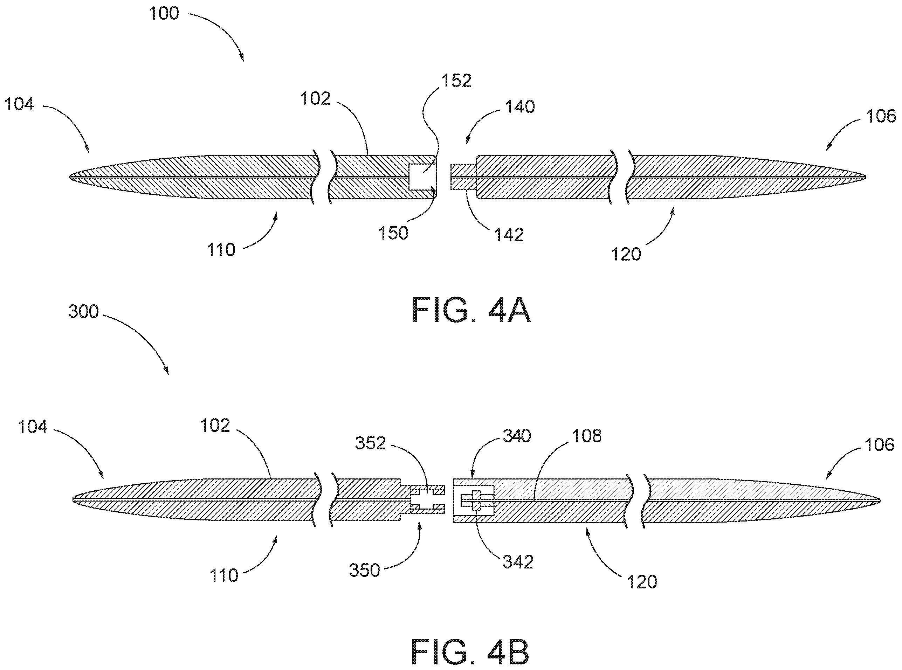

In one embodiment as shown in Figures 4A and 4B, the introducer 100 includes a proximal component 120 that defines the proximal tapered tip 106, and a distal component 110 that defines the distal tapered tip 104. The proximal component 120 and the distal component 110 are configured to be coupled together to define the introducer body 102 of the introducer. The proximal component defines a proximal portion of the bore 108. The distal component defines a distal portion of the bore 108 that is aligned with the proximal portion of the bore when the proximal and distal components are coupled together.

The introducer body 102 defines an outer surface 122 and outer cross-sectional dimension C that is perpendicular to the central axis 9. As illustrated, the outer cross-sectional dimension C does not vary along an entirety of the introducer body 102 between the proximal tapered tip 106 and the distal tapered tip 104. The proximal tapered tip 106 tapers from the outer surface 122 toward the central axis 9 along a proximal direction 4 away from the distal tapered tip 104. Furthermore, the distal tapered tip 104 tapers from the outer surface 122 toward the central axis 9 along a distal direction 2 that is opposite to the proximal direction 4. The introducer 100(or introducer body 102) is configured to be inserted through the front end 21f and into the lumen of access sheath 23 such that a movable interference fit is attained between the access sheath 23 and the introducer 100. A moveable interference fit is where the introducer can be inserted into the access sheath 23 so as to permit the introducer 100 to move through the access sheath but does it not prevent its effective use. A user can therefore exert some level of force to advance the introducer along the guidewire into and partially through the access sheath 23 while still prevent free passage of blood or other fluids between the introducer 100 and the access sheath 23. Likewise, the access sheath 23 can be removed from the introducer 100 as needed. Thus, an interference fit interferes with free passage of blood or other fuilds between the access sheath and introducer. However, a person of ordinary skill would understand that there may be some small amount of fluid loss even with such an interference fit.

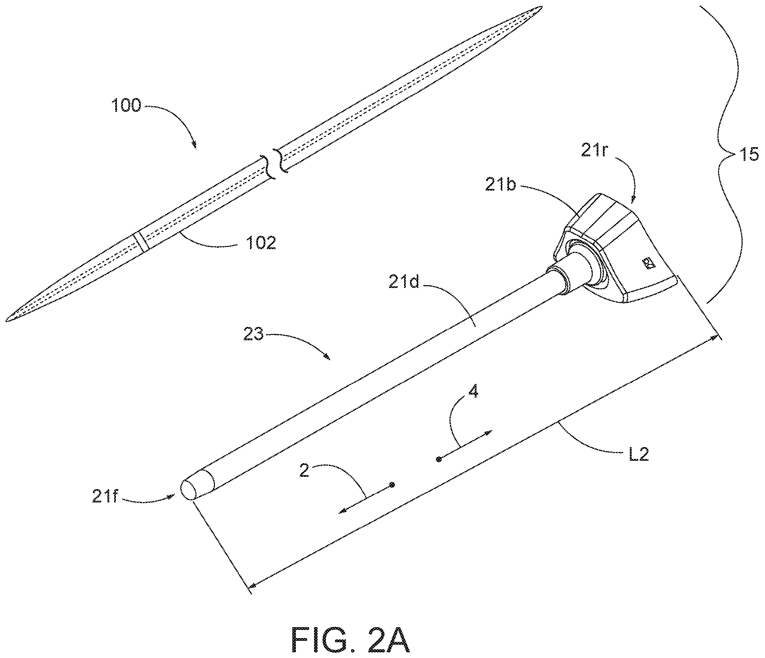

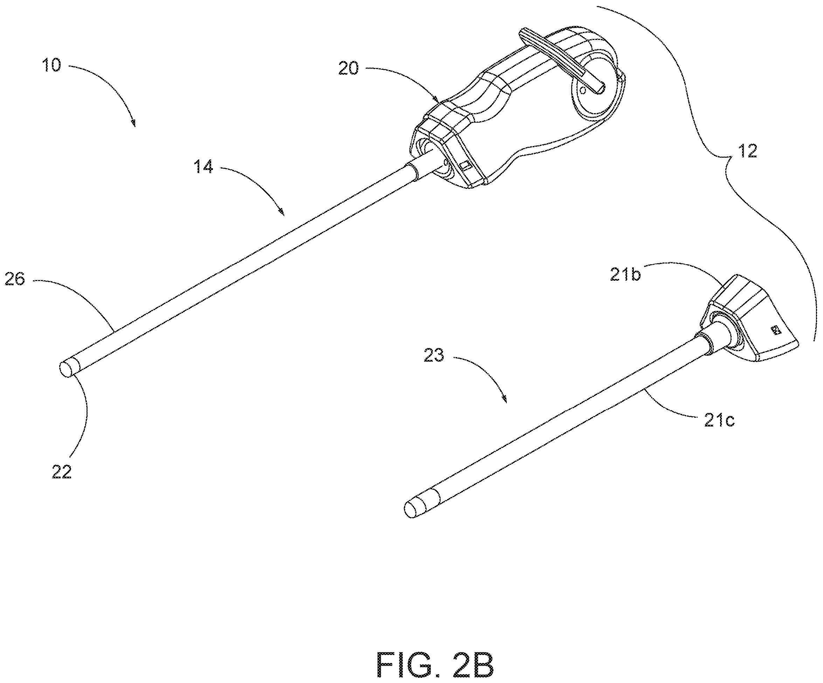

As shown in Figure 2A and 3, the introducer body 102 further defines an introducer length L1 that extends from the proximal tapered tip 160 to the distal tapered tip 104 along the central axis 9. The access sheath 23 defines a sheath length L2 that extends from the front end 21f to the rear end 21r. The length L1 of the introducer 100 is at least two times longer than the length L2 of the sheath 23. In one embodiment of the present disclosure, such when the system is used to seal puncture deep in thoracic cavity following a trans-caval procedure, the introducer length L1 is between about 120 cm and about 130 cm. In one example of such an embodiment, the introducer length is between about 123 cm and about 127 cm. In another example, the introducer length is about 125 cm. In another embodiment, such as when the system is used to seal puncture in vessel within patient's limb, the introducer length L1 can be between about 20 cm and about 30 cm. In one example of such an embodiment, the introducer length is between about 23 cm and about 27 cm. In another such example, the introducer length is about 25 cm.

As shown in Figure 3, the introducer body 102 includes at least one marker 130. Thus, there may be a single marker 130 or a plurality of markers. In an event, the marker 130 can be positioned to aid in identifying the location of the introducer 100 in the vessel. The marker 130 is one of a radio opaque band, a radio opaque ink, or a radio opaque paint. In accordance with one embodiment, a distance D1 from the distal tapered tip 104 to the marker 130 is less than a distance D2 from the marker 130 to the proximal tapered tip 106.

Referring to Figures 3-4C, the proximal component 120 and the distal component 110 are configured to be coupled together. In accordance with the illustrated embodiment, the proximal component 120 defines a first engagement member 140 opposite to the proximal tapered tip 106, and the distal component 110 defines a second engagement member 150 opposite to the distal tapered tip. The first engagement member 140 is configured to engage the second engagement member 150 so as to couple the proximal and distal components together.

The proximal component 120 and the distal component 110 are configured to be coupled together via one an interference fit. Accordingly, as illustrated in Figure 4A, the first engagement member 140 defines a projection 142, and the second engagement member 150 defines a cavity 152 sized to receive the projection 142. However, it should be appreciated that one of the first engagement member 140 and the second engagement member 150 defines a cavity, and the other of the first engagement member 140 and the second engagement member 150 defines a projection that is sized to fit in the cavity. Accordingly, either component 110, 120 may have the projection or cavity as needed.

Figure 4B illustrates another embodiment an introducer 300 that couples the proximal component and the distal component via a snap-fit connection. The introduce 300 is substantially similar to the introducer 100 described above and shown in Figure 4A. For this reason, similar reference numbers are used to identify features that are common to introducer 100 and introducer 300. The introducer 300 includes a proximal component 120 and a distal component 110. In accordance with the embodiment shown in figure 4B, a first engagement member 340 includes a ridge 342 and the second engagement member 350 includes a groove 352 that is sized to receive the ridge 342. However, it should be appreciated that one of the first engagement member 340 and the second engagement member 350 defines a ridge, and the other of the first engagement member 340 and the second engagement member 350 defines a groove that is sized to receive the ridge.

In another alternative embodiment, the proximal component and the distal component an introducer may be configured to be coupled together via a threaded connection (not shown). For instance, one of the first engagement member and the second engagement member define external threads, and the other of the first engagement member and the second engagement member define internal threads configured to threadably mate with the external threads.

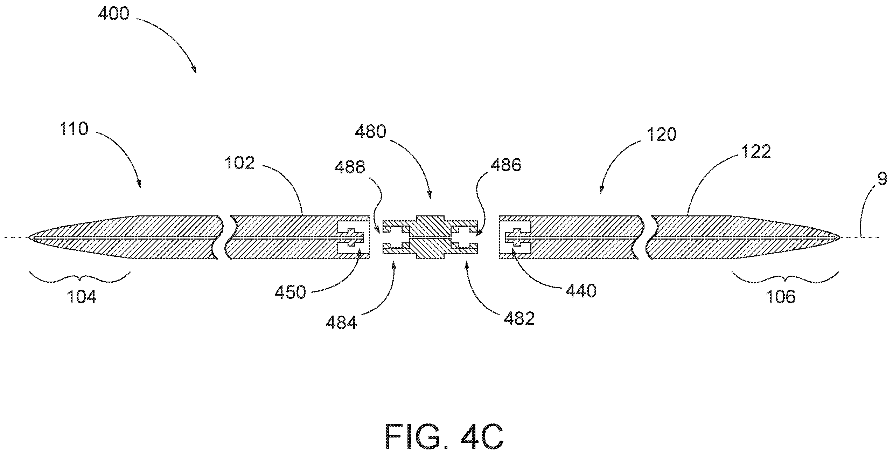

Figure 4C illustrates another embodiment an introducer 400 that couples the proximal component and the distal component via a coupler 480. The introducer 400 is substantially similar to the introducer 100 described above and shown in Figure 4A. For this reason, similar reference numbers are used to identify features that are common to introducer 100 and introducer 400. As shown in Figure 4C,the introducer 400 includes a coupler 480 having a first end 482 and a second end 484 opposite the first end 482. The first end 482 is configured to be coupled to the proximal component 120 and the second end 484 is configured to be coupled to the distal component 110. The first and second ends 482 and 484 have first and coupling members 486 and 488, respectively. The first coupling member 486 can connect to the first engagement member 440 of the proximal component 120. The second coupling member 488 can connect to the second engagement member 450 of the distal component 110. The proximal component 120, the coupler 480, and the distal component 110 when coupled together can define the introducer 400. The engagement members 440, 450 and the coupling members 486,488 can be ridges, projections, tabs, or other structures or devices that can coupled parts to each other. Accordingly, the coupler 380 and ends of the distal component 110 and proximal component 120 may be configured for any type of connection, such as an interference fit, snap-fit, threaded connection, or the like.

Referring generally to Figures 3-4C, the proximal tapered tip 106 of the introducer 100 is configured to be inserted into the front end 21f and of the access sheath 23 and through the lumen of access sheath 23. The sealing element 18 extends out from the front end 21f of the access sheath when the portion of the vascular device is coupled to the hub 21b.

As shown in Figures1 and 2A, the access sheath 23 is configured to be inserted over the introducer 100 and into the vessel. The access sheath 23 includes a hub 21b and shaft 21d that extends from the hub 21b in the distal direction 2. The access sheath 23 has a front end 21f, a rear end 21r opposite to the front end 21f, and a lumen (not numbered) that extends from the front end 21f to the rear end 21r. The rear end 21r of the access sheath includes the hub 21b that is configured to be coupled to a portion of the deployment assembly 14. When sheath 23 is coupled to the deployment assembly 14, the shaft 21d extends along the release component 22 and delivery component 26 in the distal direction 2.

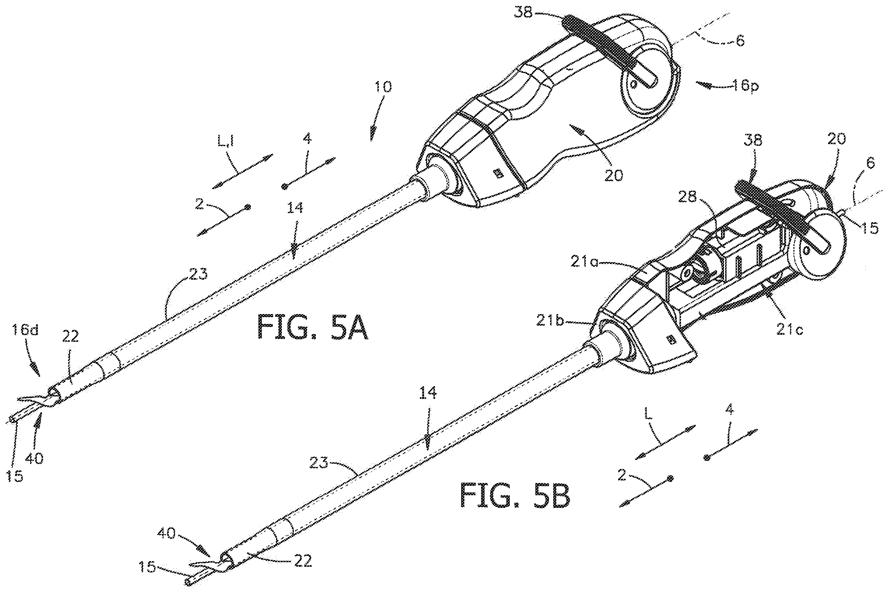

Referring to Figure 5A-5D, a vascular closure device 12 includes a sealing device or implantable unit 18 at least partially disposed within a deployment assembly 14. The vascular closure device 12 can be configured such that after a distal portion of deployment assembly 14 is inserted through a puncture site of the vessel, the sealing device 18 is deployed to thereby seal or otherwise close the puncture site of the vessel. The deployment assembly 14 is configured to control orientation of a toggle 40 of the sealing device 18 in an easier and more efficient manner during deployment of the sealing device 18. Furthermore, the deployment assembly 14 is configured to reduce forces required to deploy the sealing device 18 and seal the puncture.

In accordance with the illustrated embodiment, the deployment assembly 14 includes a release component 22 that restrains the toggle 40, a delivery component 26 that contains at least a portion of the toggle 40 and a suture 44 of the sealing device 18, a guide member 35, and one or more actuators 38 coupled to the release component 22. The deployment assembly 14 may also include a tamper 70, in the form a tube, that extend along the suture 44 is a located proximal with respect to the locking member 230 (See Figure 5D). The guide member 35 extends through the sealing device 18 and is configured to receive a guidewire 35 as will be discussed below. In another example, the deployment assembly 14 can be configured so that the guidewire 35 extends along the side of the toggle 40. The release component 22 is operatively associated with the suture 44 such that actuation of the actuator 38 causes the release component 22 to 1) release the toggle 40, and 2) apply tension to the suture 44, which urges the toggle 40 against the delivery component 26 and orients the toggle 40 in the sealing position. The guide member 35 is configured to be removed from at least the sealing device 18 prior the sealing device 18 sealing the puncture.

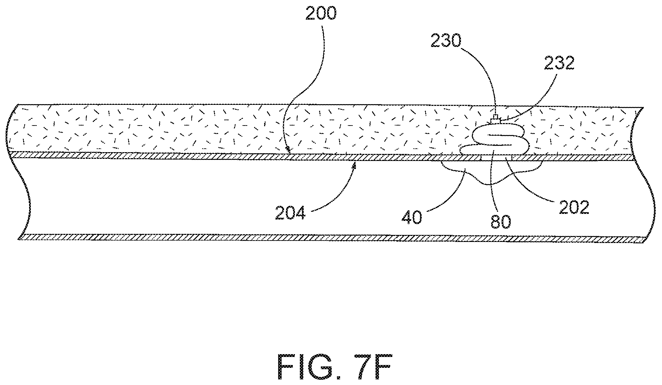

Turning to Figure 5C, the sealing device 18 includes the toggle 40 connected to the suture 44, a plug 88 coupled to the suture 44 and spaced from the toggle 40 in a proximal direction 4, and a locking member 230 proximal to the plug 88. The toggle 40 includes a distal end 45d and a proximal end 41p opposite to the proximal end 41p, and a plurality of apertures (not numbered) extending therethrough. The suture 44 extends through the apertures as illustrated such that an end of the suture 44 is formed into a slidable knot 232. The knot 232 is slidable along the suture 44 between the plug 88 and the locking member 230. In an implanted state, the toggle 40 is adjacent an inner surface of the vessel and the locking member 230 squeezes the toggle 40 the plug 88 against the vessel to seal the puncture. See for example Figure 7F.

The sealing device 18 is formed with materials suitable for surgical procedures such as any biocompatible material. For example, the toggle 40 can be made of a polylacticcoglycolic acid or other synthetic absorbable polymer that degrades in the presence of water into naturally occurring metabolites. In other embodiments, the toggle can be made of stainless steel, biocorrodible iron, and biocorrodible magnesium. It should be appreciated, however, that the toggle 40 can be made of other materials and can have other configurations so long as it can be seated inside the vessel against the vessel wall. The plug 88 can comprise a strip of compressible, resorbable, collagen foam and can be made of a fibrous collagen mix of insoluble and soluble collagen that is cross linked for strength. It should be appreciated, however, that the plug member 88 can have any configuration as desired and can be made from any material as desired. The suture 44 can be any elongate member, such as, for example a filament, thread, or braid.

Referring again Figures 5A, 5B and 5D, the deployment assembly 14 is elongate along a longitudinal direction L and includes a rear end 16p and a front end 16d spaced from the rear end 16p along an axis 6 that is aligned with the longitudinal direction L. The longitudinal direction L can include and define a distal direction 2 that extends from the rear end 16p toward the front end 16d. Further, the longitudinal direction L can include and define a proximal direction 4 that is opposite the distal direction 2 and that extends from front end 16d toward the rear end 16p. The deployment assembly 14 is configured to insert the toggle 40 into the vessel along an insertion direction I (see Figure 4). The longitudinal direction L can be aligned with the insertion direction I during a portion of the sealing procedure.

Turning to Figures 5A and 5B, in accordance with the illustrated embodiment, the deployment assembly 14 includes a handle member 20, the release component 22 supported by the handle manner 20 and extending from handle member 20 in the distal direction 2, the delivery component 26 also supported by the handle member 20 and extending along the distal direction 2, and a tensioner 28 supported by the handle member 20 and positioned adjacent to the release component 22. A portion of delivery component 26 is shown in dashed lines in Figure 5A and 5B.

The actuator 38 is coupled to both the handle member 20 and the release component 22. As noted above the actuator 38 is configured to 1) cause the release component 22 to move in the proximal direction 4 from a first or initial position relative to the delivery component 26 into a second or releasing position relative to the delivery component 26, and 2) apply a tensile force to the suture 44 during or subsequent to movement of the release component 22 from the initial position into the released position. The description below refers to the release component 22 being moveable relative to the delivery component 26. But the deployment assembly 14 can be configured so that the delivery component 26 is moveable relative to the release component 22. The deployment assembly 14 also includes the guide member 35 that extends through the deployment assembly 14, and an outer sheath 23 that contains and supports portions of the release component 22 and delivery component 26. Furthermore, the actuator 38 may be adapted to operate, or cause move the tamper 70 along the suture 44 to tamp the sealing unit into a tamped, deployed configuration. In alternative embodiment, an separate actuator may be used to control the tamper 70.

Continuing with Figures 5A and 5B, the handle member 20 includes a housing 21a and a cavity 21c defined at least partly by housing 21a and a nose 21b of the access sheath 23. The cavity 21c is sized to contain a portion of the release component 22, delivery component 26, and the tensioner 28.

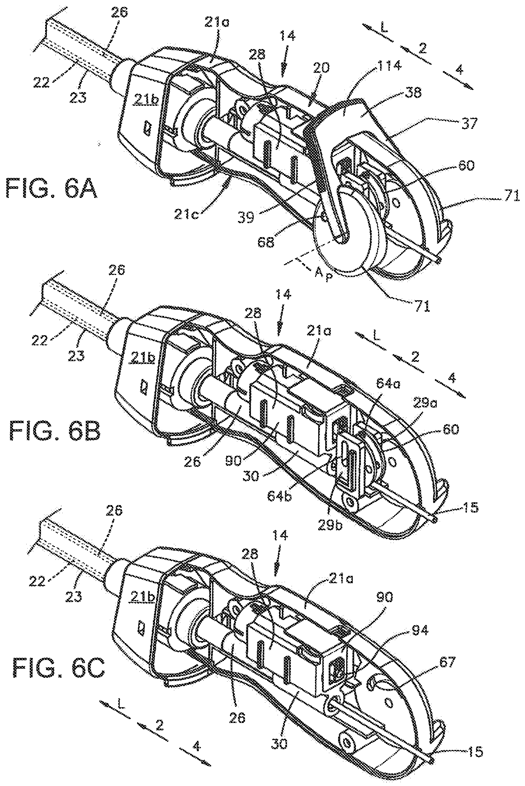

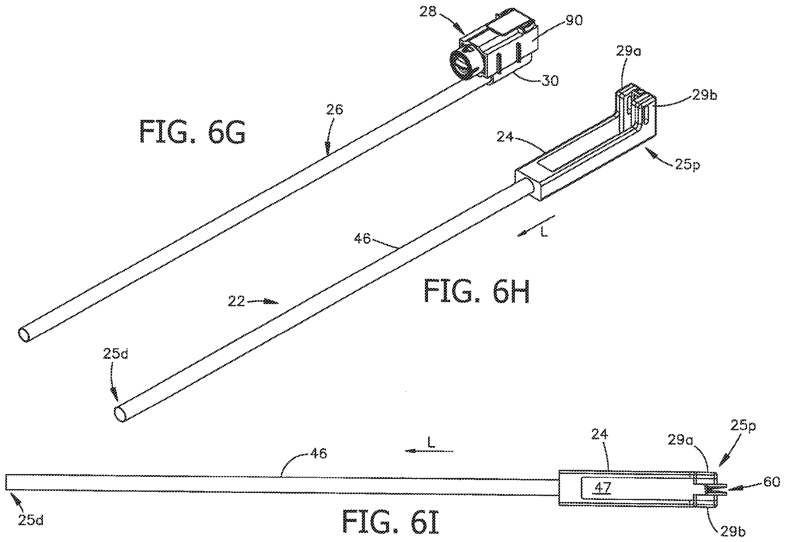

Turning to Figures 5B, 6H and 6I, the release component 22 is elongate along a first or longitudinal direction L defines a distal end 25d and a proximal end 25p spaced from the distal end 25d along the longitudinal direction L. In accordance with the illustrated embodiment, the release component 22 includes a release hub 24 and a release tube 46 that is fixed to the release hub 24 extends from the release hub 24 in the distal direction 2. The release hub 24 includes a pair of tabs 29a, 29b disposed at the proximal end 25p of the release component 22. A pulley 60 is coupled to the tabs 29a, 29b and defines a curved track that receives the suture 44 as will be explained below. The hub 24 defines a slot 47 that is elongate along the longitudinal direction L and is aligned with the release tube 46. The slot 47 is sized to receiver a coupler 30 of the tensioner 28.

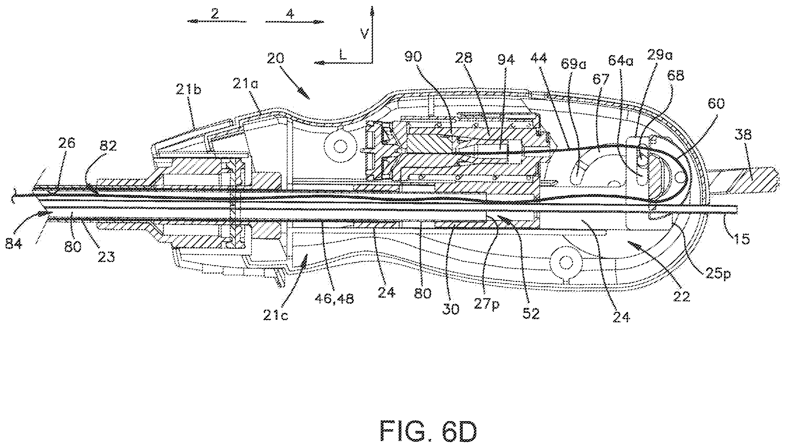

The release tube 46 includes a release tube body 48 that is elongate along the longitudinal direction L. The release tube body 48 defines a release tube channel 52 that extends along the longitudinal direction L from the hub 24 toward the proximal end 25p. In the illustrated embodiment, the release tube channel 52 (Figure 6D) extends completely through the release tube body 48 from the hub 24 to the distal end 25d. Furthermore, in the illustrated embodiment the release tube body 48 is cylindrical such that the release tube channel 52 is radially enclosed. It should be appreciated, however, that the release tube channel 52 can extend partially through the release tube body 48 as desired and that the release tube body 48 can have other configurations as desired. For example, the release tube body 48 can be U-shaped such that the release tube channel 52 is partially radially open. As shown, the release tube channel 52 is sized to slidably receive a portion of the delivery component 26 such that the release component 22 is movable relative to the delivery component 26.

Referring to Figures 6A, 6B, 6D, and 6I, the pulley 60 is disposed at the proximal end 25p of the release component 22. As shown, the suture 44 extends around the pulley 60 along the guide track and into the tensioner 28. As the release component 22 is pulled in the proximal direction 4, the pulley 60 pulls the suture 44 in proximal direction 4 thereby applying a tensile force to the toggle 40. In such an embodiment, the tensioner 28 is positioned alongside the release component 22. It should be appreciated, however, that in some embodiments, the tensioner 28 is positioned proximal to the release tube and is in-line with the release component 22 such that the suture 44 extends through the release tube and into the tensioner 28 along the first direction L.

With continued reference to 6A, 6B, 6D, and 6I, the release component 22 can include at least one mating member 64 that mates with a corresponding mating member 68 of the actuator 38 to thereby transfer the motion of the actuator 38 to the release component 22. In the illustrated embodiment, the release component mating member 64 is a pair of slots 65a and 65b defined by the respective pair of tabs 29a and 29b. Each slot 65a and 65b is elongate along a direction a vertical direction V that is perpendicular to the first direction L. The actuator 38 mating member 68 can be operatively engaged with elongate slots 65a and 65b of release component 22 such that actuation of the actuator 38 causes the release component 22 to translate along the first direction L. It should be appreciated, however, that the mating member 64 can have any configuration as desired. For example, the mating member 64 can be a bore having a diameter that is equal to that of the pin such that translation of the actuator 38 along the first direction L causes the release component 22 to translate along the first direction L.

As shown in Figures 5B, 6D-6G, the delivery component 26 is coupled to the tensioner 28 and extends along the release component 22 toward the front end 16d of the deployment assembly 14. In accordance with the illustrated embodiment, because the tensioner 28 is fixed to the housing 21a, the delivery component 26 is fixed to the housing 21a and thus the handle member 20. The delivery component 26 includes a delivery tube body 80 that is elongate along the first direction L and defines a distal end 27d and a proximal end 27p spaced from the distal end 27d in the first direction L. The delivery tube body 80 defines an inner surface 81, which in turns defines a delivery tube channel 84 that extends at least partially through the delivery tube body 80 along the first direction L. As illustrated embodiment, the delivery tube channel 84 extends completely through the delivery tube body 80 from the proximal end 27p to the distal end 27d. However, the channel 84 can extend along a portion of the delivery tube body 80. Furthermore, in the illustrated embodiment the delivery tube body 80 is cylindrical such that the delivery tube channel 84 is radially enclosed. It should be appreciated, however, that the delivery tube channel 84 can extend partially through the delivery tube body 80 as desired and that the delivery tube body 80 can have other configurations as desired. For example, the delivery tube body 80 can be U-shaped such that the delivery tube channel 84 is partially radially open. As illustrated, the proximal end 27p of delivery component is fixed to the tensioner 28 and the distal end 27d of delivery component is configured to hold at least a portion of the sealing device 18 (Figure 5D).

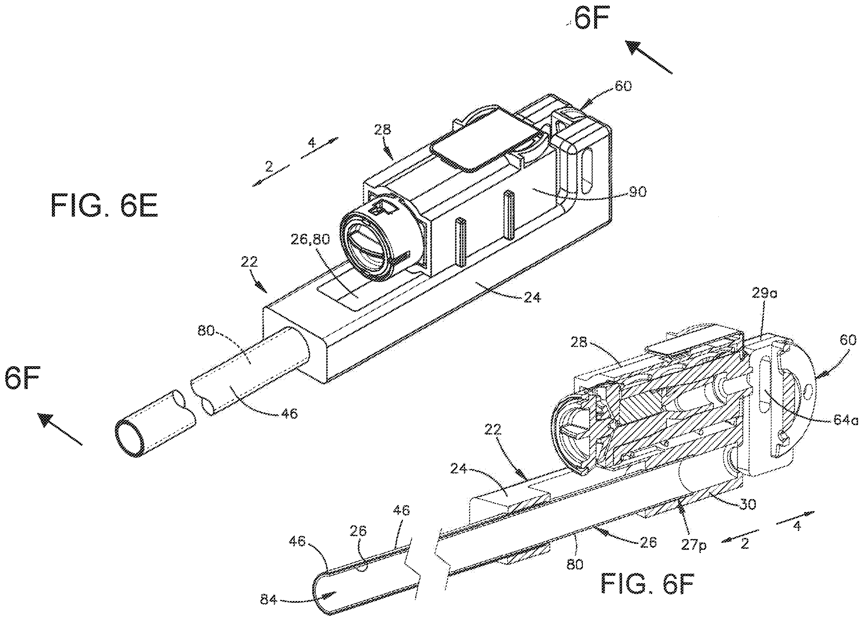

The delivery tube channel 84 is sized to retain at least a portion of the sealing device 18. In particular, the plug 88 and locking member 230 are retained within the delivery tube channel 84, while the toggle 40 is configured to be initially trapped between the delivery component 26 and the release component 22. For instance, the distal end 25d of the release tube 48 defines an offset surface 49, which can be angled with respect to the longitudinal axis 6. The offset surface 49 and inner surface 81 of the delivery tube 80 define a cavity 51 that receives the proximal end 41p of the toggle 40 when release component 22 is in the initial position (as shown in Figure 5D). The angle of the offset surface 49 can define the orientation of the toggle 40 in this initial position, whereby the distal end 45D of the toggle 40 is spaced some distance in the distal direction 2 beyond the distal ends 25d and 27d of the release component 22 and delivery component 26, respectively. The suture 44 extends from the toggle 40 through the delivery tube channel 84, through the proximal end 27p (Figure 6D) around the pulley 60 along the guide track and is coupled to the tensioner 28. The guide member 35 extends through the channel 84 and exits the front end 16d of the vascular closure device 12. When the actuator 38 is actuated as will be further detailed below, the release component 22 moves in the proximal direction 4 thereby releasing the proximal end 41p of the toggle 40 from between the release component 22 and the delivery component 26. As the release component 22 moves in the proximal direction 4, the suture 44 will be pulled in the proximal direction 4 to thereby place the suture 44 in tension and urge the toggle 40 against the distal end 27d of the delivery component 26. At this point, the toggle 40 is oriented in the sealing position (see Figure 6E). In the sealing position, the toggle 40 has been repositioned so that the toggle 40 is placed against the distal end 27d of the delivery component 26 and is oriented more transversely with respect to the axis 6 compared to the position when the toggle 40 is restrained by the release component 22.

As shown in Figures 6D-6G, the tensioner 28 disposed on the delivery component 26 and is positioned alongside the release component 22 so as to receive the suture 44 as noted above. In accordance with the illustrated embodiment, the tensioner 28 includes a tensioner housing 90, a coupler 30 that extends from the housing 90 and is attached to the delivery component 26, and a drag member 94 disposed within the tensioner housing 90. The suture 44 extends into the tensioner housing 90 through the drag member 94 and such that a frictional force is applied to the suture 44 by the drag member 94. The tensioner housing 90 is coupled the housing 21a and fixed thereto. The coupler 30 as illustrated is a tubular component that receives the proximal end 27p of the delivery tube body 80. As illustrated, the delivery tube body 80 is fixed to the coupler 30 which indirectly fixes the delivery component 26 to the housing 21A.

The suture 44 extends from the proximal end 27p of the delivery tube body 80, through the coupler 30, around the pulley 60 and into the drag member 94 and is spooled within the tensioner housing 90 (not shown). Spooling the suture 44 in tensions housing 90 allows suture 44 to dispends from the deployment assembly 14 when the deployment assembly 14 is pulled is proximal direction 2 to thereby deploy the sealing device 18 (see Figures 6F and 6G). The frictional force applied to the suture 44 by the drag member 94 can be high enough to maintain the suture 44 in tension after the actuator 38 has been actuated and the toggle 40 has been urged against the distal end 27d of the delivery component 26. At the same time the frictional force applied to the suture 44 by the drag member 94 can be low enough to allow the suture 44 to dispense from the tensioner housing 90 when the deployment assembly 14 is pulled in a proximal direction 4 relative to the toggle 40. In the illustrated embodiment, the drag member 94 is a silicon member that pinches the suture 44. The tensioner 90 and drag member 94 can be similar the tensioner described in U.S. Patent Application Publication No. 2013/0178895. It should be appreciated, however, that the drag member 94 can have other configurations as desired.

Turning to Figures 6A-6D, the deployment assembly 14 can include one or more actuators that are configured to transition the release component 22 into to releasing position and to cause a tension to be applied to suture 44 when toggle 40 is released from the release component 22 as described above. As noted above, the actuator 38 can include the mating member 68 that operatively engages the mating member 64 of the release component 22 such that motion of the actuator 38 relative to the handle member 20 causes the release component to translate in the proximal direction 4 and further applies a tension to the filament.

In accordance with the illustrated embodiment, the actuator 38 can be configured as a lever that is rotatably coupled to the handle member 20. The actuator 38 or lever can include a pair of side members 71a and 71 rotatably coupled to each side of the housing 21a, a first leg 37a that extends from one of the side members 71a, a second leg 37b that extends from the other side member 71b, and a transverse member 39 that connects the first leg 37a to the second leg 37b. The actuator 38 is configured to pivot about a pivot axis A_{P} that is perpendicular to the axis 6. The pivot axis A_{P} may or may not intersect axis 6. The housing 21a defines a curved housing slot 67 that is curved with respect to the pivot axis A_{P}. The curved housing slot 67 has a first end 69a (Fig. 6D) and second end (not numbered) spaced apart from the first end along the proximal direction 4. The mating member 68 of the actuator 38 can be a pin 68 that is coupled to and extends between the side members 71 and 71b at a location that is offset from the pivot axis A_{P}. The pin 68 extends through curved housing slot 67 and through the elongate slots 64a and 64a of the hub 24 of the release component 22 such that the actuator 38 is operatively coupled to the release component 22.

In use, as the actuator 38 pivots about the pivot axis A_{P}, the pin 68 moves from the first end 69a the curved housing slot 67 toward the second end of the curved housing slot 67, and also moves along the slots 64a and 64b along the vertical direction V. Because the release component 22 is moveable relative to housing 21a, as pin 68 moves along the curved housing slot 67, the pin 68 advances the hub 24 of the release component 22 in the proximal direction 4. The result in accordance with the illustrated embodiment is that rotation of the actuator 38 causes the release component 22 to translate in the longitudinal direction L. It should be appreciated, however, that the actuator 38 can have other configurations as desired and is not limited to the disclosed lever.

In operation, the deployment assembly 14 is initially configured to insert the toggle 40 into the vessel. When the actuator 38 is actuated, the release component 22 moves in the proximal direction 4 relative to the delivery component 26 into the releasing position (not illustrated) thereby releasing the proximal end 41p of the toggle 40 from between the release component 22 and the delivery component 26. As the release component 22 moves in the proximal direction 4, the suture 44 will be pulled in the proximal direction 4 to thereby place the suture 44 in tension and urge the toggle 40 against the distal end 27d of the delivery component 26. At this point, the toggle 40 is oriented in the sealing position (see Figure 6E). Accordingly, the release component 22 is configured to restrain the toggle 40 of the sealing device 18 during insertion of the vascular closure device 12 into the vessel and subsequently release the toggle 40 so that the toggle 40 can be oriented for the sealing procedure.

The release component 22 is also in communication the suture 44 via the pulley 60 such that when the actuator 38 is actuated the release component 22 pulls the suture 44 in the proximal direction to thereby place the suture 44 in tension. Application of tension along the suture 44 urges the toggle 40 against the distal end 27d of the delivery component 26 and orients the toggle 40 into the sealing position. In the illustrated embodiment, the actuator 38 and release component 22 are configured such that continuous movement of the actuator 38 relative to the housing 21a will move the release component 22 in the proximal direction 4, thereby releasing the toggle 40 from the release component 22 and subsequently apply tension to the suture 44. It should be appreciated, however, that in some embodiments the suture 44 can be tensioned as the toggle 40 is being released. It should further be appreciated that in some embodiments, the deployment assembly 14 can include a first actuator to release the toggle 40 and a second actuator that tensions the suture 44.

The release component 22 and delivery components 26 are described above has having tubular shaped bodies. It should be appreciated that the release and delivery components can have other configurations. For instance, the release component can be elongate rod, or an elongate rod with a tubular ring coupled to its distal end. The delivery component can be configured such that only a portion thereof has a tubular shape.

Embodiments of the present technology will now be described with respect to exemplary large bore procedures that utilize the vascular closure system 100 illustrated in Figures 7A-7F. In order to perform any of the related procedures, the user gains percutaneous access to, for example, the femoral artery, causing a puncture site in the artery. To gain percutaneous access to the artery, the Seldinger technique may be used. For example, a hollow bore needle is inserted into the vessel 200 through a procedure sheath PS (referred to as the first access sheath). A guidewire is then advanced through the hollow needle into the femoral artery a sufficient distance to allow removal of the needle without the guidewire pulling out of the vessel. Removing the needle leaves the guidewire in place, with a portion of the guidewire extending into the artery and proximal end PE of the sheath PS extending out patient. The guidewire, extending from outside the patient into the femoral artery, provides for an entry guide for other medical devices including the access sheath 23, introducer 100, and vascular closure device 12. Therefore, once the guidewire is positioned in the vessel of the patient, catheters, or introducers, of gradually increasing diameters are advanced over the guidewire and through the puncture into the artery to further open the puncture site. Then, a procedure access sheath set (*i.e.* an introducer 100 inside a procedure sheath PS) is moved along the guidewire such that a distal end DE of the sheath PS moves into the vessel through the puncture site. And once positioned, the introducer can be removed such that the sheath provides for sizable access to the vessel interior from outside the body. After the relevant procedure is completed, the puncture site in the artery created during percutaneous access of the artery may be closed. The vascular closure system may be used to seal the puncture site.

In some instances, however, access through the femoral artery as described above is not indicated due to condition of the vessel between the femoral artery and the aorta. In such cases, a trans-caval procedure can be used to access the aorta. As shown in Figure 7A, the trans-caval procedure includes guiding a guidewire 35 through into through a first puncture 202 in a femoral vein 200 and further into and a portion of the inferior vena cava 210. The method include creating a second puncture 212 in the portion of the inferior vena cava 210 and creating a third puncture 222 in a femoral artery 220 and a portion of the aorta 220. The punctures can formed with a tip of the guidewire 35, such as by burning. Next, the distal end DE of the procedure access sheath PS is guided along the guidewire 35 through the second and third punctures. When the sheath PS is in place, a medical device, such as a catheter, is inserted through the first access sheath PS. When the procedure is completed, the catheter is removed from the procedure access sheath PS and the guidewire 35.

Continuing with Figure 7A, a method includes positioning a tapered distal end 104 of an introducer over a proximal end 36 of a guidewire 35 that extends through a puncture 202 in a vessel 200, e.g. a vena cava, such that the guidewire 35 enters a bore 108 of the introducer. In the example illustrated, the procedure sheath PS and guidewire 35 extends from the outside the patient into the femoral venal cava.

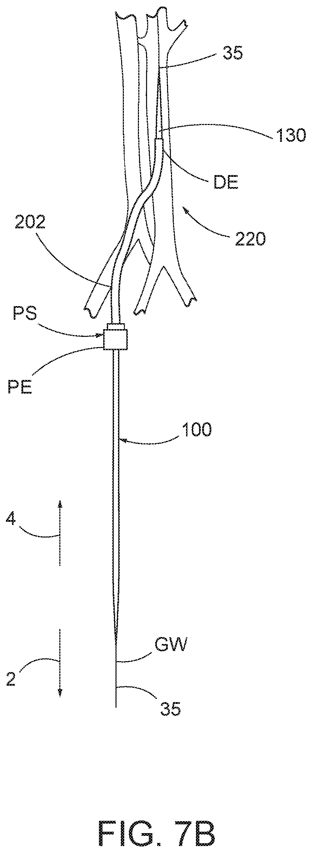

Next, as shown in Figure 7B, the method includes advancing the introducer 100along the guidewire 35 in a distal direction 4 so that the tapered distal end enters the proximal end PE of the first access sheath PS. The introducer 100 further advanced out of a distal end DE of the access sheath PS that is spaced from the proximal end PE of the access sheath PS in the distal direction 4. The introducer 100 is advanced in the distal direction until a marker 130 disposed toward the tapered distal end is positioned within a predetermined distance of the puncture of the vessel.

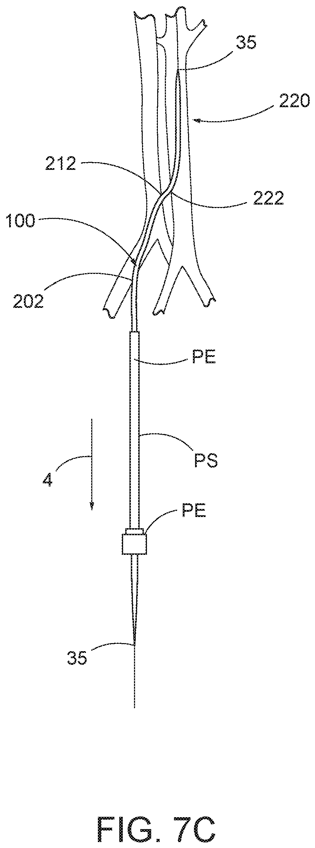

As shown in Figure 7B and 7C, the method includes removing the access sheath PS from the punctures 202, 212 and 214 while maintaining a portion of the tapered distal end 104 of the introducer 100 in the aorta 220 (or some other vessel as the case may be).

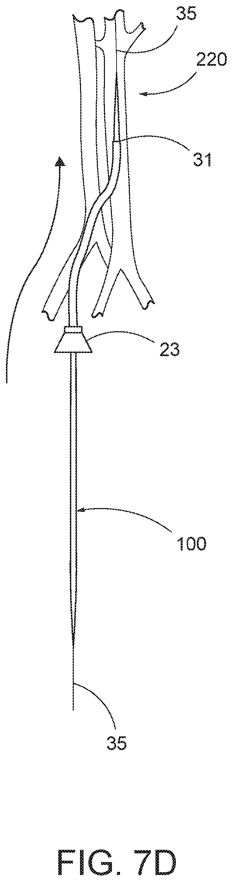

As shown in Figure 7D, after the removing step, the procedure includes the step of inserting the access sheath 23 of system 10 (also referred to as the second access sheath) over the tapered proximal end of the introducer until a distal or front end 31 of the access sheath 23 extends through the puncture of the vessel.

As shown in Figure 7D, the method includes the step of removing the introducer 100 from the access sheath 23 and the guidewire 35. The exchange of sheaths PS and 23 limits blood loss and ensure smooth transition between the interventional procedure and sealing the puncture site.

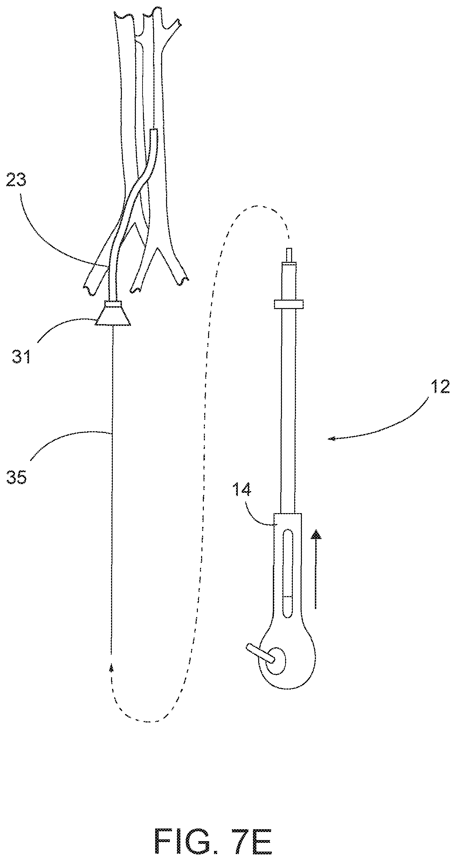

As shown in Figure 7E, the method includes advancing a vascular closure device, for instance the deployment assembly 14, into the access sheath 23 to seal the puncture. In one example, the method can also include the steps of sealing the puncture 222, sealing puncture 212 and sealing puncture 202. In most cases, the sealing device 18 is deployed as illustrated in Figure 7F. As deployed, the toggle 40 is adjacent the vessel wall 204, the plug 88 is collapses against the outer surface of the wall 204 and opposite the toggle 40. The knot 232 and lock member 230 secure the plug 88 in place, compressing the plug 88 and toggle 40 together.

It should be appreciated that the introducer 100 can be assembled during manufacture or at the surgical site prior to its use as described above. The method can include coupling a distal component 110 of the introducer 100 to a proximal component 120 of the introducer, wherein the distal component 110 defines the tapered distal end 104 and the proximal component 120 defines the tapered proximal end 106.

The method described above is related to a trans-caval procedure. It should be appreciated that the vascular closure system can be used to seal punctures in a femoral artery. In particular, the vascular closure system may be used to see so-call large bore punctures, such as 10F French, 12 French, 14 French or larger sized bore. Such a system is typically used to seal a puncture in vessel within patient's limb.

## Claims

1. A system configured to seal a puncture in a vessel, comprising:
an introducer (100) configured to be inserted through the puncture along a guidewire (35) that extends through the puncture into the vessel, the introducer including an introducer body (102) that is elongate along a central axis (9), a proximal tapered tip (106), a distal tapered tip (104) that is opposite to the proximal tapered tip (106) along the central axis (9), and a bore (108) that extends from the proximal tapered tip (106) to the distal tapered tip (104) along the central axis (9), the bore (108) sized to receive the guidewire (35) therethrough, wherein the distal tapered tip (104) is configured to be inserted into the vessel along the guidewire (35);
an access sheath (23) configured to be inserted over the introducer (100) and into the vessel, the access sheath (23) including a front end (21f) , a rear end (21r) opposite to the front end (21f), and a lumen that extends from the front end (21f) to the rear end (21r), wherein the proximal tapered tip (106) of the introducer (100) is configured to be inserted through the front end (21f) and into the lumen of access sheath (23) such that an interference fit allowing relative movement between the access sheath (23) and the introducer (100) is attained; and
a vascular closure device (12) including a sealing element (18) configured to seal the puncture of the vessel, wherein when the introducer (100) has been removed from the lumen of the access sheath (23), the rear end (21r) of the access sheath (23) is configured to receive the vascular closure device (12) such that the sealing element extends out of the front end (21f) of the access sheath (23).

2. The system of claim 1, wherein the introducer (100) includes a proximal component (120) that defines the proximal tapered tip (106), and a distal component (110) that defines the distal tapered tip (104), wherein the proximal and distal components (120, 110) are configured to be coupled together to define the introducer body (102).

3. The system of claim 2, wherein the proximal component (120) defines a proximal portion of the bore (108) and the distal component (110) defines a distal portion of the bore (108) that is aligned with the proximal portion of the bore (108) when the proximal and distal components (120, 110) are coupled together.

4. The system of claim 2, wherein the proximal component (120) and the distal component (110) are configured to be coupled together via one of a) an interference fit, b) a snap-fit connection, or c) a threaded connection.

5. The system of claim 2, wherein the proximal component (120) defines a first engagement member (140) opposite to the proximal tapered tip (106), and the distal component (110) defines a second engagement member (150) opposite to the distal tapered tip (104), wherein the first engagement member (140) is configured to engage the second engagement member (150) so as to couple the proximal and distal components (120, 110) together.

6. The system of claim 5, wherein one of the first engagement member (140) and the second engagement member (150) define external threads, and the other of the first engagement member (140) and the second engagement member (150) define internal threads configured to threadably mate with the external threads.

7. The system of claim 5, wherein one of the first engagement member (140) and the second engagement member (150) defines a ridge (342), and the other of the first engagement member (140) and the second engagement member (150) defines a groove (352) that is sized to receive the ridge (342).

8. The system of claim 2, further comprising a coupler (480) having a first end (482) and a second end (484) opposite the first end (482), the first end (482) configured to be coupled to the proximal component (120) and the second end (484) configured to be coupled to the distal component (110), such that the proximal component (120), the coupler (480), and the distal component (110) define the introducer (100).

9. The system of claim 1, wherein the introducer body (102) defines an outer cross-sectional dimension (C) that is perpendicular to the central axis (9), wherein the outer cross-sectional dimension (C) does not vary along an entirety of the introducer body (102) between the proximal tapered tip (106) and the distal tapered tip (104).

10. The system of claim 1, wherein the introducer body (102) defines an outer surface (122), and the proximal tapered tip (106) tapers from the outer surface (122) toward the central axis (9) along a proximal direction (4) away from the distal tapered tip (104), wherein the proximal direction (4) is aligned with the central axis (9).

11. The system of claim 10, wherein the distal tapered tip (104) tapers from the outer surface (122) toward the central axis (9) along a distal direction (2) that is opposite to the proximal direction (4).

12. The system of claim 1, wherein the introducer body (102) further defines an introducer length (L1) that extends from the proximal tapered tip (106) to the distal tapered tip (104) along the central axis (9), and the access sheath (23) defines a sheath length (L2) that extends from the front end (21f) to the rear end (21r), wherein the introducer length (L1) is at least two times longer than the sheath length (L2).

13. The system of claim 1, wherein the introducer body (102) further defines an introducer length (L2) that extends from the proximal tapered tip (106) to the distal tapered tip (104) along the central axis (9), wherein the introducer length (L2) is between about 120 cm and about 130 cm.

14. The system of claim 13, wherein the introducer length (L2) is between about 123 cm and about 127 cm.

15. The system of claim 14, wherein the introducer length (L2) is about 125 cm.

16. The system of claim 1, wherein the introducer body (102) further defines an introducer length (L2) that extends from the proximal tapered tip (106) to the distal tapered tip (104) along the central axis (9), wherein the introducer length (L2) is between about 20 cm and about 30 cm.

17. The system of claim 16, wherein the introducer length (L2) is between about 23 cm and about 27 cm.

18. The system of claim 17, wherein the introducer length (L2) is about 25 cm.

19. The system of claim 1, wherein the introducer body (102) includes at least one marker (130).

20. The system of claim 19, wherein a distance (D1) from the distal tapered tip (104) to the at least one marker (150) is less than a distance (D2) from the at least one marker (130) to the proximal tapered tip (106).

21. The system of claim 19, wherein the at least one marker (130) is one of a radio opaque band, a radio opaque ink, or a radio opaque paint.

22. The system of claim 1, wherein the rear end (21r) of the access sheath (23) includes a hub (21b) that is configured to be coupled to a portion of the vascular closure device (12), wherein the sealing element extends out from the front end (21f) of the access sheath (23) when the portion of the vascular device (12) is coupled to the hub (21b).

## Patentansprüche

1. System, konfiguriert zum Abdichten einer Punktion in einem Gefäß, umfassend:
ein Einführinstrument (100), das dazu konfiguriert ist, durch die Punktion entlang eines Führungsdrahtes (35), der sich durch die Punktion in das Gefäß erstreckt, eingesetzt zu werden, wobei das Einführinstrument einen Einführinstrumentenkörper (102), der länglich entlang einer Mittelachse (9) ausgebildet ist, eine proximale, verjüngte Spitze (106), eine distale, verjüngte Spitze (104), die der proximale, verjüngten Spitze (106) entlang der Mittelachse (9) gegenüberliegt, und einen Kanal (108), der sich von der proximale, verjüngten Spitze (106) zu der distale, verjüngten Spitze (104) entlang der Mittelachse (9) erstreckt, wobei der Kanal (108) so bemessen ist, dass er den Führungsdraht (35) hindurch aufnehmen kann, wobei die distale, verjüngte Spitze (104) dazu konfiguriert ist, entlang des Führungsdrahtes (35) in das Gefäß eingesetzt zu werden;
eine Zugangsschleuse (23), die dazu konfiguriert ist, über das Einführinstrument (100) und in das Gefäß eingesetzt zu werden, wobei die Zugangsschleuse (23) ein vorderes Ende (21f), ein hinteres Ende (21r), das dem vorderen Ende (21f) gegenüberliegt, und ein Lumen, das sich von dem vorderen Ende (21f) zu dem hinteren Ende (21r) erstreckt, beinhaltet, wobei die proximale, verjüngte Spitze (106) des Einführinstruments (100) dazu konfiguriert ist, durch das vordere Ende (21f) und in das Lumen der Zugangsschleuse (23) eingesetzt zu werden, so dass eine Presspassung erreicht wird, die eine Relativbewegung zwischen der Zugangsschleuse (23) und dem Einführinstrument (100) ermöglicht; und
eine Gefäßverschlussvorrichtung (12), die ein Dichtungselement (18) beinhaltet, das dazu konfiguriert ist, die Punktion des Gefäßes abzudichten, wobei das hintere Ende (21r) der Zugangsschleuse (23) dazu konfiguriert ist, die Gefäßverschlussvorrichtung (12) aufzunehmen, wenn das Einführinstrument (100) aus dem Lumen der Zugangsschleuse (23) entfernt wurde, sodass sich das Dichtungselement über das vordere Ende (21f) der Zugangsschleuse (23) hinaus erstreckt.

2. System nach Anspruch 1, wobei das Einführinstrument (100) eine proximale Komponente (120), die die proximale, verjüngte Spitze (106) definiert, und eine distale Komponente (110), die die distale, verjüngte Spitze (104) definiert, beinhaltet, wobei die proximale und die distale Komponente (120, 110) dazu konfiguriert sind, miteinander gekoppelt zu werden, um den Einführinstrumentenkörper (102) zu definieren.

3. System nach Anspruch 2, wobei die proximale Komponente (120) einen proximalen Abschnitt des Kanals (108) definiert und die distale Komponente (110) einen distalen Abschnitt des Kanals (108) definiert, der mit dem proximalen Abschnitt des Kanals (108) ausgerichtet ist, wenn die proximale und die distale Komponente (120, 110) miteinander gekoppelt sind.

4. System nach Anspruch 2, wobei die proximale Komponente (120) und die distale Komponente (110) dazu konfiguriert sind, über eines von a) einer Presspassung, b) einer Schnappverbindung oder c) einer Gewindeverbindung miteinander gekoppelt zu werden.

5. System nach Anspruch 2, wobei die proximale Komponente (120) ein erstes Eingriffselement (140) gegenüber der proximalen, verjüngten Spitze (106) definiert und die distale Komponente (110) ein zweites Eingriffselement (150) gegenüber der distalen, verjüngten Spitze (104) definiert, wobei das erste Eingriffselement (140) dazu konfiguriert ist, in das zweite Eingriffselement (150) einzugreifen, um die proximale und die distale Komponente (120, 110) miteinander zu koppeln.

6. System nach Anspruch 5, wobei eines von dem ersten Eingriffselement (140) und dem zweiten Eingriffselement (150) ein Außengewinde definiert und das andere von dem ersten Eingriffselement (140) und dem zweiten Eingriffselement (150) ein Innengewinde definiert, das dazu konfiguriert ist, mit dem Außengewinde verschraubt zu werden.

7. System nach Anspruch 5, wobei eines von dem ersten Eingriffselement (140) und dem zweiten Eingriffselement (150) einen Vorsprung (342) definiert und das andere von dem ersten Eingriffselement (140) und dem zweiten Eingriffselement (150) eine Nut (352) definiert, die bemessen ist, den Vorsprung (342) aufzunehmen.

8. System nach Anspruch 2, ferner umfassend einen Koppler (480), der ein erstes Ende (482) und ein zweites Ende (484), das dem ersten Ende (482) gegenüberliegt, aufweist, wobei das erste Ende (482) dazu konfiguriert ist, mit der proximalen Komponente (120) gekoppelt zu werden, und das zweite Ende (484) dazu konfiguriert ist, mit der distalen Komponente (110) gekoppelt zu werden, sodass die proximale Komponente (120), der Koppler (480) und die distale Komponente (110) das Einführinstrument (100) definieren.

9. System nach Anspruch 1, wobei der Einführinstrumentenkörper (102) eine äußere Querschnittsabmessung (C) definiert, die senkrecht zu der Mittelachse (9) verläuft, wobei sich die äußere Querschnittsabmessung (C) entlang einer Gesamtheit des Einführinstrumentenkörpers (102) zwischen der proximalen, verjüngten Spitze (106) und der distalen, verjüngten Spitze (104) nicht ändert.

10. System nach Anspruch 1, wobei der Einführinstrumentenkörper (102) eine Außenfläche (122) definiert und sich die proximale, verjüngte Spitze (106) von der Außenfläche (122) in Richtung der Mittelachse (9) entlang einer proximalen Richtung (4) weg von der distalen, verjüngten Spitze (104) verjüngt, wobei die proximale Richtung (4) mit der Mittelachse (9) ausgerichtet ist.

11. System nach Anspruch 10, wobei sich die distale, verjüngte Spitze (104) von der Außenfläche (122) in Richtung der Mittelachse (9) entlang einer distalen Richtung (2) verjüngt, die der proximalen Richtung (4) entgegengesetzt ist.

12. System nach Anspruch 1, wobei der Einführinstrumentenkörper (102) ferner eine Einführinstrumentenlänge (L1) definiert, die sich von der proximalen, verjüngten Spitze (106) entlang der Mittelachse (9) zu der distalen, verjüngten Spitze (104) entlang der Mittelachse (9) erstreckt, und die Zugangsschleuse (23) eine Schleusenlänge (L2) definiert, die sich von dem vorderen Ende (21f) zu dem hinteren Ende (21r) erstreckt, wobei die Einführinstrumentenlänge (L1) mindestens zweimal länger als die Schleusenlänge (L2) ist.

13. System nach Anspruch 1, wobei der Einführinstrumentenkörper (102) ferner eine Einführinstrumentenlänge (L2) definiert, die sich von der proximalen, verjüngten Spitze (106) entlang der Mittelachse (9) zu der distalen, verjüngten Spitze (104) erstreckt, wobei die Einführinstrumentenlänge (L2) zwischen etwa 120 cm und etwa 130 cm beträgt.

14. System nach Anspruch 13, wobei die Einführinstrumentenlänge (L2) zwischen etwa 123 cm und etwa 127 cm beträgt.

15. System nach Anspruch 14, wobei die Einführinstrumentenlänge (L2) etwa 125 cm beträgt.

16. System nach Anspruch 1, wobei der Einführinstrumentenkörper (102) ferner eine Einführinstrumentenlänge (L2) definiert, die sich von der proximalen, verjüngten Spitze (106) entlang der Mittelachse (9) zu der distalen, verjüngten Spitze (104) erstreckt, wobei die Einführinstrumentenlänge (L2) zwischen etwa 20 cm und etwa 30 cm beträgt.

17. System nach Anspruch 16, wobei die Einführinstrumentenlänge (L2) zwischen etwa 23 cm und etwa 27 cm beträgt.

18. System nach Anspruch 17, wobei die Einführinstrumentenlänge (L2) etwa 25 cm beträgt.

19. System nach Anspruch 1, wobei der Einführinstrumentenkörper (102) mindestens eine Markierung (130) beinhaltet.

20. System nach Anspruch 19, wobei ein Abstand (D1) von der distalen, verjüngten Spitze (104) zu der mindestens einen Markierung (150) geringer als ein Abstand (D2) von der mindestens einen Markierung (130) zu der proximalen, verjüngten Spitze (106) ist.

21. System nach Anspruch 19, wobei die mindestens eine Markierung (130) eines von einem strahlendichten Band, einer strahlendichten Tinte oder einer strahlendichten Farbe ist.

22. System nach Anspruch 1, wobei das hintere Ende (21r) der Zugangsschleuse (23) eine Nabe (21b) aufweist, die dazu konfiguriert ist, mit einem Abschnitt der Gefäßverschlussvorrichtung (12) gekoppelt zu werden, wobei sich das Dichtungselement über das vordere Ende (21f) der Zugangsschleuse (23) hinaus erstreckt, wenn der Abschnitt der Gefäßvorrichtung (12) mit der Nabe (21b) gekoppelt ist.

## Revendications

1. Un système configuré pour obturer une ponction dans un vaisseau, comprenant :
un dispositif d'introduction (100) configuré pour être inséré à travers la ponction le long d'un fil-guide (35) qui se prolonge à travers la ponction jusqu'à l'intérieur du vaisseau, le dispositif d'introduction comprenant un corps de dispositif d'introduction (102) s'étendant de façon allongée le long d'un axe central (9), une extrémité conique proximale (106), une extrémité conique distale (104) opposée à l'extrémité conique proximale (106) le long de l'axe central (9), et un canal (108) se prolongeant de l'extrémité conique proximale (106) jusqu'à l'extrémité conique distale (104) le long de l'axe central (9), le canal (108) étant dimensionné pour recevoir le fil-guide (35), l'extrémité conique distale (104) étant configurée pour être insérée dans le vaisseau le long du fil-guide (35) ;
une gaine d'accès (23) configurée pour être insérée sur le dispositif d'introduction (100) et dans le vaisseau, la gaine d'accès (23) comprenant une extrémité avant (21f), une extrémité arrière (21r) opposée à l'extrémité avant (21f), et une lumière se prolongeant de l'extrémité avant (21f) jusqu'à l'extrémité arrière (21r), l'extrémité conique proximale (106) du dispositif d'introduction (100) étant configurée pour être insérée à travers l'extrémité avant (21f) et dans la lumière de la gaine d'accès (23) de telle sorte qu'un ajustement par interférence permettant un mouvement relatif entre la gaine d'accès (23) et le dispositif d'introduction (100) soit obtenu ; et
un dispositif de fermeture vasculaire (12) comprenant un élément d'obturation (18) configuré pour obturer la ponction du vaisseau, dans lequel, lorsque le dispositif d'introduction (100) a été retiré de la lumière de la gaine d'accès (23), l'extrémité arrière (21r) de la gaine d'accès (23) est configurée pour recevoir le dispositif de fermeture vasculaire (12), de telle sorte que l'élément d'obturation se prolonge hors de l'extrémité avant (21f) de la gaine d'accès (23).

2. Le système selon la revendication 1, dans lequel le dispositif d'introduction (100) comprend une composante proximale (120) définissant l'extrémité conique proximale (106), et une composante distale (110) définissant l'extrémité conique distale (104), dans lequel les composantes proximale et distale (120, 110) sont configurées pour être assemblées ensemble de manière à définir le corps du dispositif d'introduction (102).

3. Le système selon la revendication 2, dans lequel la composante proximale (120) définit une partie proximale du canal (108) et la composante distale (110) définit une partie distale du canal (108), cette dernière étant alignée avec la partie proximale du canal (108) lorsque les composantes proximale et distale (120, 110) sont assemblées.

4. Le système selon la revendication 2, dans lequel la composante proximale (120) et la composante distale (110) sont configurées pour être assemblées ensemble au moyen, au choix, a) d'un ajustement par interférence, b) d'un assemblage encliquetable, ou c) d'un assemblage fileté.

5. Le système selon la revendication 2, dans lequel la composante proximale (120) définit un premier organe d'engagement (140) situé à l'opposé de l'extrémité conique proximale (106), et la composante distale (110) définit un second organe d'engagement (150) situé à l'opposé de l'extrémité conique distale (104), dans lequel le premier organe d'engagement (140) est configuré pour entrer en contact avec le second organe d'engagement (150) de manière à assembler les composantes proximale et distale (120, 110).

6. Le système selon la revendication 5, dans lequel l'un parmi le premier organe d'engagement (140) et le second organe d'engagement (150) définit des filets externes et l'autre parmi le premier organe d'engagement (140) et le second organe d'engagement (150) définit des filets internes configurés pour s'emboîter par filetage avec les filets externes.

7. Le système selon la revendication 5, dans lequel l'un parmi le premier organe d'engagement (140) et le second organe d'engagement (150) définit une saillie (342), et l'autre parmi le premier organe d'engagement (140) et le second organe d'engagement (150) définit une rainure (352) dimensionnée pour recevoir la saillie (342).

8. Le système selon la revendication 2, comprenant également un élément de liaison (480) présentant une première extrémité (482) et une seconde extrémité (484) opposée à la première extrémité (482), la première extrémité (482) étant configurée pour être assemblée à la composante proximale (120) et la seconde extrémité (484) étant configurée pour être assemblée à la composante distale (110), de telle sorte que la composante proximale (120), l'élément de liaison (480) et la composante distale (110) définissent le dispositif d'introduction (100).

9. Le système selon la revendication 1, dans lequel le corps du dispositif d'introduction (102) définit une dimension de section transversale externe (C) perpendiculaire à l'axe central (9), dans lequel la dimension de section transversale externe (C) ne varie pas sur l'ensemble du corps du dispositif d'introduction (102), entre l'extrémité conique proximale (106) et l'extrémité conique distale (104).

10. Le système selon la revendication 1, dans lequel le corps du dispositif d'introduction (102) définit une surface externe (122), et l'extrémité conique proximale (106) est effilée depuis la surface externe (122) vers l'axe central (9) selon une direction proximale (4) orientée à l'opposé de l'extrémité conique distale (104), dans laquelle la direction proximale (4) est alignée avec l'axe central (9).

11. Le système selon la revendication 10, dans lequel l'extrémité conique distale (104) est effilée depuis la surface externe (122) vers l'axe central (9) selon une direction distale (2) opposée à la direction proximale (4).

12. Le système selon la revendication 1, dans lequel le corps du dispositif d'introduction (102) définit également une longueur d'introduction (L1) se prolongeant de l'extrémité conique proximale (106) à l'extrémité conique distale (104) le long de l'axe central (9), et la gaine d'accès (23) définit une longueur de gaine (L2) se prolongeant de l'extrémité avant (21f) à l'extrémité arrière (21r), dans lequel la longueur d'introduction (L1) est au moins deux fois supérieure à la longueur de gaine (L2).

13. Le système selon la revendication 1, dans lequel le corps du dispositif d'introduction (102) définit également une longueur d'introduction (L2) se prolongeant de l'extrémité conique proximale (106) à l'extrémité conique distale (104) le long de l'axe central (9), dans lequel la longueur d'introduction (L2) est comprise entre environ 120 cm et environ 130 cm.

14. Le système selon la revendication 13, dans lequel la longueur d'introduction (L2) est comprise entre environ 123 cm et environ 127 cm.

15. Le système selon la revendication 14, dans lequel la longueur d'introduction (L2) est d'environ 125 cm.

16. Le système selon la revendication 1, dans lequel le corps du dispositif d'introduction (102) définit également une longueur d'introduction (L2) qui se prolonge de l'extrémité conique proximale (106) à l'extrémité conique distale (104) le long de l'axe central (9), dans lequel la longueur d'introduction (L2) est comprise entre environ 20 cm et environ 30 cm.

17. Le système selon la revendication 16, dans lequel la longueur d'introduction (L2) est comprise entre environ 23 cm et environ 27 cm.

18. Le système selon la revendication 17, dans lequel la longueur d'introduction (L2) est d'environ 25 cm.

19. Le système selon la revendication 1, dans lequel le corps du dispositif d'introduction (102) comprend au moins un marqueur (130).

20. Le système selon la revendication 19, dans lequel une distance (D1) entre l'extrémité conique distale (104) et l'au moins un marqueur (150) est inférieure à une distance (D2) entre l'au moins un marqueur (130) et l'extrémité conique proximale (106).

21. Le système selon la revendication 19, dans lequel l'au moins un marqueur (130) est l'un des éléments suivants : une bague radio-opaque, une encre radio-opaque ou une peinture radio-opaque.

22. Le système selon la revendication 1, dans lequel l'extrémité arrière (21r) de la gaine d'accès (23) comprend un embout (21b) configuré pour être assemblé à une partie du dispositif de fermeture vasculaire (12), dans lequel l'élément d'obturation se prolonge hors de l'extrémité avant (21f) de la gaine d'accès (23) lorsque la partie du dispositif de fermeture vasculaire (12) est assemblée à l'embout (21b).
